# EUROPÄISCHE PATENTANMELDUNG

(11) **EP 1 112 991 A2**
(43) Veröffentlichungstag der Anmeldung: **04.07.2001**
(21) Anmeldenummer: 00127639.3
(22) Anmeldetag: 15.12.2000
(51) Int. Cl.: C07C 17/26, C07C 22/08, C07C 51/16, C07C 51/265, C07C 63/331

(54) **Verfahren zur Herstellung von 4'-Trifluormethyl-2-methylbiphenyl und 4'-Trifluormethylbiphenyl-2-carbonsäure aus o-Tolylmetallaten**

(30) Priorität: 29.12.1999 DE 19963562
(71) Anmelder: Clariant GmbH, 65929 Frankfurt am Main (DE)
(72) Erfinder: Meudt, Andreas, Dr., 65439 Flörsheim-Weilbach (DE); Scherer, Stefan, Dr., 64572 Büttelborn (DE); Nörenberg, Antje, Dr., 64572 Büttelborn (DE); Koch, Peter, Dr., 60318 Frankfurt/Main (DE); Haber, Steffen, Dr., 76829 Landau/Pfalz (DE)

(57) **Zusammenfassung**

Die Erfindung betrifft ein Verfahren zur Herstellung einer Verbindung der Formel (I) 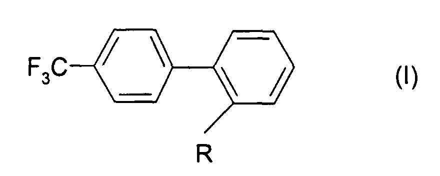 worin R die Bedeutung Methyl oder Carboxyl hat, dadurch gekennzeichnet, dass man ein ortho-Tolylmetallat der Formel (II) mit einer Verbindung der Formel (III) 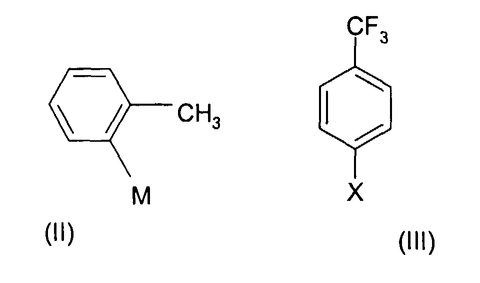 worin
M die Bedeutung -MgF, -MgCI, -MgBr, -Mgl, -Li, -ZnF, -ZnCI, -ZnBr oder -Znl und
X die Bedeutung F, Cl, Br, I, N₂⁺, geradkettiges oder verzweigtes (C₁-C₂₀)-Alkoxy, Arylsulfonat oder Alkylsulfonat hat,
in Gegenwart eines Ni-, Pd- oder Platinmetall-Katalysators zu einer Verbindung der Formel (I) mit R gleich CH₃ kuppelt, und gegebenenfalls die Verbindung der Formel (I) mit R gleich CH₃ zur Verbindung der Formel (I) mit R gleich Carboxyl oxidiert.

## Beschreibung

Die Erfindung betrifft ein neues Verfahren zur Herstellung von Verbindungen der Formel (I) 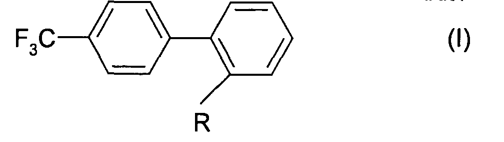 worin R die Bedeutung Methyl oder Carboxyl hat.
Trifluormethylbiphenyl-2-carbonsäure ist ein wichtiges Zwischenprodukt in der Synthese von Wirkstoffen der pharmazeutischen Industrie. Aufgrund der hohen Bedeutung ist eine Reihe von Synthesen literaturbeschrieben, die jedoch alle durch spezifische wirtschaftliche und verfahrenstechnische Nachteile gekennzeichnet sind.

In der US-A-4 578 522 und EP-A-0 059 983 ist die Kupplung von p-Trifluormethylphenylmagnesiumbromid mit Dimethyloxazolinanisol und nachfolgende saure Hydrolyse des Oxazolinrestes beschrieben; die Gesamtausbeute beträgt hierbei 28,4 % (Kupplung 33 %; Hydrolyse 86 %). Nachteilig sind hier neben den unbefriedigenden Ausbeuten die Verwendung der Oxazolin-Schutzgruppe für die Säurefunktion.

Ein zweites Verfahren (US-A-4 578 522, EP-A-0 059 983) kuppelt die Grignardverbindung des o-Brombenzaldehyddimethylacetals mit p-lodbenzotrifluorid unter Palladiumkatalyse. Die Ausbeute beträgt 86 % nach Chromatographie bezogen auf den lodaromaten. In einem zweiten Schritt wird der Aldehyd mittels KMnO₄ zur Carbonsäure oxidiert (Ausbeute 85 %). Nachteilig ist hier vor allem der Einsatz der schwer zugänglichen Brom- bzw. lodaromaten.

Eine dritte Variante (J. Org. Chem. 1977, 42, 1821; J. Labelled Compd. and Radiopharm. 1992, 21, 2011) geht von Phenyldimethyloxazolin aus, das mit n-Butyllithium selektiv deprotoniert, mit Zinkchlorid transmetalliert und unter Pd-Katalyse mit p-lodbenzotrifluorid gekuppelt wird. Nach saurer Spaltung des Oxazolins erhält man das Zielmolekül.

Ein weiteres Verfahren kuppelt p-Chlorbenzotrifluorid unter Nickel-Katalyse mit der ortho-Zinkverbindung des Phenyldimethyloxazolins (Org. Prep. Proced. Int. 1995, 27 (3), 367). Man erhält hier die Zielverbindung in einer Ausbeute von 64 % bezogen auf p-Chlorbenzotrifluorid.

In diesen beiden Verfahren werden die recht guten Ausbeuten durch eine umständliche und aufwendige Synthese in ihrer Bedeutung gemindert. Allen beschriebenen Verfahren ist die Verwendung der Oxazolin-Schutzgruppe und die damit verbundenen Nachteile aus atomökonomischer und verfahrenstechnischer Sicht gemeinsam.

Die Herstellung von 4'-Trifluormethyl-2-methylbiphenyl durch Kreuzkupplung wird von A. Indolese, Tetrahedron Lett. 38, 20, 1997, 3513-3516 beschrieben. Die Kupplung von o-Tolylboronsäure mit p-Chlorbenzotrifluorid unter Katalyse mit NiCl₂(dppf) in Dioxan führt nur zu einer Ausbeute von 26 % an 4'-Trifluormethyl-2-methylbiphenyl.

Der Erfindung lag die Aufgabe zugrunde, ein industriell durchführbares Verfahren zur Herstellung von 4'-Trifluormethyl-2-methyl-biphenyl und 4'-Trifluormethyl-biphenyl-2-carbonsäure bereitzustellen, das frei von den oben genannten Nachteilen ist und vor allem die Verwendung teurer Schutzgruppen wie der Oxazolin-Schutzgruppe umgeht. Des weiteren sollte das zu entwickelnde Verfahren möglichst frei von technisch aufwendigen Reinigungsprozeduren wie Umkristallisation oder Chromatographie sein und das Produkt in einer für die Herstellung pharmazeutischer Wirkstoffe erforderlichen hohen Reinheit liefern.

Überraschenderweise wurde gefunden, dass 4'-Trifluormethylbiphenyl-2-carbonsäure verfahrenstechnisch einfach, in guten Ausbeuten und in hoher Reinheit durch ein zweistufiges Verfahren erhältlich ist, indem man a) o-Tolylmetallate der Formel (II) mit in 4-Stellung eine geeignete Abgangsgruppe X tragenden Trifluormethylbenzolen der Formel (III) kuppelt und b) das so erhältliche 4'-Trifluormethyl-2-methylbiphenyl durch geeignete Oxidationsmittel zur entsprechenden Carbonsäure oxidiert.

Stufe a):

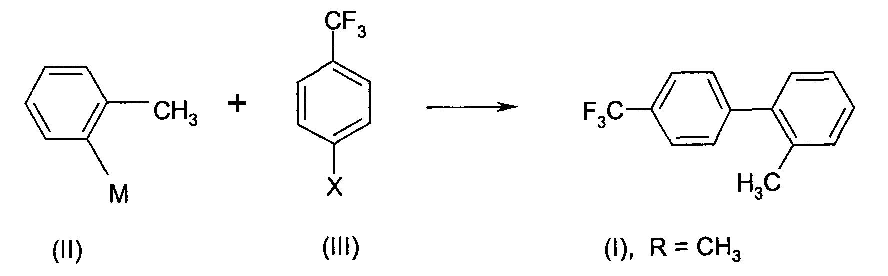

Stufe b):

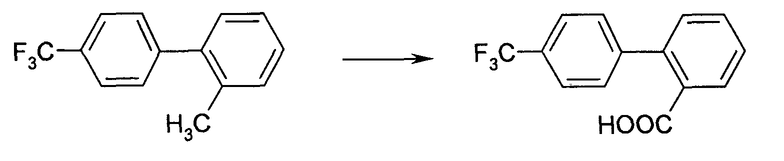

Gegenstand der vorliegenden Erfindung ist ein Verfahren zur Herstellung einer Verbindung der Formel (I) 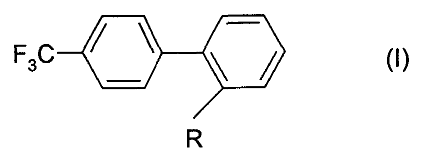 worin R die Bedeutung Methyl oder Carboxyl hat, dadurch gekennzeichnet, dass man ein ortho-Tolylmetallat der Formel (II) mit einer Verbindung der Formel (III) 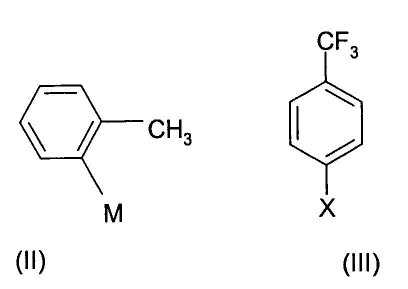 worin
M die Bedeutung -MgF, -MgCI, -MgBr, -Mgl, -Li, -ZnF, -ZnCI, -ZnBr, -Znl und X die Bedeutung F, Cl, Br, I, N₂⁺, geradkettiges oder verzweigtes (C₁-C₂₀)-Alkoxy, Arylsulfonat oder Alkylsulfonat hat,
in Gegenwart eines Ni-, Pd- oder Platinmetall-Katalysators zu einer Verbindung der Formel (I) mit R gleich CH₃ kuppelt, und gegebenenfalls die Verbindung der Formel (I) mit R gleich CH₃ zur Verbindung der Formel (I) mit R gleich Carboxyl oxidiert.

Bevorzugte o-Tolylmetallate sind o-Tolylmagnesiumchlorid und -bromid.

Bevorzugte Verbindungen der Formel (III) sind solche, in denen X Chlor, Brom, Triflat, Tosylat, Nonaflat, Mesylat bedeutet, besonders bevorzugt ist 4-Chlortrifluor-methylbenzol.

Geeignete Katalysatoren für die Kupplungsreaktionen sind in der Literatur beschrieben und dem Fachmann geläufig. Bevorzugt sind hierbei Salze, Komplexe oder die metallische Form von Nickel, Palladium oder einem anderen Platinmetall, wie z.B. Rh und Pt.

Besonders bevorzugt sind Salze oder Komplexe von Nickel oder Palladium sowie metallische Formen, ggf. auf einem geeigneten Träger, z.B. Pd auf C oder auf BaSO₄, ganz besonders bevorzugt PdCl₂(dppf), PdCl₂(PPh₃)₂, PdCl₂(dppe), PdCl₂(dppp), PdCl₂(dppb), Pd(PPh₃)₄, Pd(OAc)2, PdCl₂, PdBr₂ oder NiCl₂(PPh₃)₂, worin "dppf" 1,2-Bis(diphenylphosphino)ferrocen, "dppe" 1,2-Bis(diphenyl-phosphino)ethan, "dppp" 1,2-Bis(diphenylphosphino)propan, "dppb" 1,2-Bis-(diphenylphosphino)butan, "Ph" Phenyl und "Ac" Acetyl bedeuten.

Die Mengen des Katalysators können zwischen 0,00001 und 50 mol-%, vorzugsweise 10⁻⁴ und 10 Mol-%, bezogen auf Verbindung der Formel (III), liegen.

Die Kupplung wird zweckmäßigerweise in Lösungsmitteln, wie beispielsweise aliphatischen oder aromatischen Ethern, aromatischen oder aliphatischen Kohlenwasserstoffen, halogenierten aromatischen oder aliphatischen Kohlenwasserstoffen oder Mischungen der genannten Lösungsmittel durchgeführt, bevorzugt in THF oder THF/Toluol-Gemischen.

Zweckmäßige Reaktionstemperaturen liegen je nach Aktivität des Katalysators und der Substrate zwischen 0°C und 180°C, bevorzugt zwischen 30°C und 150°C, besonders bevorzugt zwischen 50 und 100°C.

Die molaren Mengenverhältnisse zwischen der Verbindung der Formel (II) und (III) sind zweckmäßig (II):(III) 0,85:1 bis 1,2:1.

Unter den erfindungsgemäßen Bedingungen wird überraschenderweise die Entstehung von Dimeren, nämlich 2,2'-Dimethylbiphenyl und 4,4'-Bis(trifluormethyl)biphenyl, durch Homokupplung fast vollständig verhindert.

Es hat sich als vorteilhaft erwiesen, die Lösung oder Suspension des o-Tolylmetallates zu einer Lösung des substituierten Trifluormethylbenzols und des Katalysators zu dosieren. Ebenso ist es aber auch möglich, das Trifluormethylbenzol oder eine Lösung des Trifluormethylbenzols in einem Lösungsmittel zur Lösung oder Suspension des o-Tolylmetallates zu dosieren, oder eine Mischung der Reaktanden zur Katalysatorlösung zu tropfen.

Die Aufarbeitung geschieht zweckmäßigerweise durch Eintragen der ausreagierten Reaktionslösung in Wasser oder eine wässrige Lösung einer geeigneten Säure, bevorzugt Salzsäure oder Schwefelsäure, wobei Temperaturen zwischen 0 und 80°C und Säurekonzentrationen zwischen 0 und 35 Gew.-% bevorzugt sind. Das in der organischen Phase gelöste 4'-Trifluormethyl-2-methylbiphenyl kann durch Destillation in hochreiner Form erhalten werden.

Die Oxidation des 4'-Trifluormethyl-2-methylbiphenyls kann durch Oxidationsmittel, wie Salpetersäure, Permanganat, Chrom(VI)-Verbindungen, Sauerstoff oder Luft, geschehen. Bevorzugt sind hierbei Kaliumpermanganat, Natriumpermanganat oder Luft, besonders bevorzugt Luft.

Die Oxidation mit Kaliumpermanganat kann sowohl in wässriger Lösung als auch in nichtwässrigem Medium geschehen. In wässriger Lösung ist die Gegenwart geeigneter Phasentransferkatalysatoren, beispielsweise Tetraalkylammoniumsalzen, Tetraphenylphosphoniumsalzen oder Kronenethern, für die Erzielung guter Ausbeuten förderlich. Die Aufarbeitung geschieht durch Filtration des entstandenen Braunsteins, Ansäuern und Abfiltrieren der 4'-Trifluormethylbiphenyl-2-carbonsäure.

Die Oxidation mit Luft wird in aliphatischen Carbonsäuren, gegebenenfalls im Gemisch mit Wasser, durchgeführt. Bevorzugt wird Essigsäure verwendet, besonders bevorzugt ist ein Gemisch aus Essigsäure und Wasser. Als Katalysator dienen Schwermetallsalze, z. B. Gemische aus Cobalt- und Mangansalzen, insbesondere -bromiden. Die Schwermetallsalze werden in Mengen von je 0,01 bis 5,0 Mol-%, bevorzugt 0,1 bis 4,0 Mol-%, besonders bevorzugt 1,0 bis 2,0 Mol-%, bezogen auf 4'-Trifluormethyl-2-methyl-biphenyl, eingesetzt. Das Verhältnis zwischen Cobalt- und Mangansalzen lässt sich in weiten Grenzen variieren und kann beispielsweise zwischen 1:5 und 5:1 liegen. Bevorzugt werden die beiden Salze äquimolar eingesetzt. Die Reaktion wird bei Temperaturen zwischen 100 und 200°C, bevorzugt zwischen 120 und 180°C und besonders bevorzugt zwischen 150 und 170°C durchgeführt. Der Druck liegt zwischen Normaldruck und 50 bar. Die Aufarbeitung geschieht durch Abkühlen und gegebenenfalls Einengen der Reaktionsmischung, Filtration, Waschen und Trocknung der entstandenen ausgefallenen Carbonsäure. Im vorliegenden Fall erhält man ein Gemisch aus der gewünschten 4'-Trifluormethylbiphenyl-2-carbonsäure und dem daraus unter den Reaktionsbedingungen gebildeten Fluorenon der Formel (V)

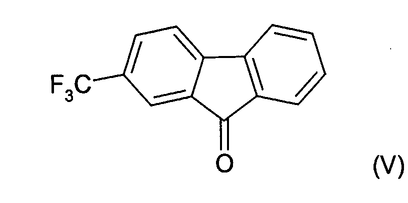

Die gewünschte Biphenylcarbonsäure lässt sich aus diesem Gemisch in einfacher Weise durch Extraktion mit Alkalien und anschließendes Ausfällen mit Mineralsäuren in reiner Form isolieren.

Beispiele zur Herstellung von 4'-Trifluormethyl-2-methylbiphenyl

### Beispiel 1

### Kupplung von o-Tolylmagnesiumchlorid mit p-Trifluormethylchlorbenzol

Zu einer siedenden Lösung von 993 g p-Trifluormethylchlorbenzol (5,5 mol) und Palladium(II)chlorid(dppf) (4,1 g; 0,1 mol-%) in 1316 g THF wurde in 2 Stunden eine 26 gew.-%ige Lösung von o-Tolylmagnesiumchlorid (5,0 mol) in THF dosiert. Nach sechsstündiger Reaktionszeit am Siedepunkt der Mischung (73°C) erfolgte die wässrige Aufarbeitung mit 600 ml 0,1 gew.-%iger Schwefelsäure. Das Produkt konnte durch fraktionierte Vakuumdestillation (105°C/8 mbar) rein erhalten werden; die Ausbeute betrug 940 g (94,7 %).

Die Verringerung der Katalysatormenge auf 100 mg (0,0023 mol-%) und gleichzeitige Erhöhung der Reaktionszeit auf 11 Stunden führte zu einer ähnlichen Ausbeute.

### Beispiel 2

### Kupplung von o-Tolylmagnesiumbromid mit p-Trifluormethylchlorbenzol

Die Kupplung wurde analog zu der in Beispiel 1 beschriebenen Reaktion ausgeführt (0,01 mol-% PdCl₂(dppf); 6 Stunden). Die Ausbeute betrug 93,2 %.

### Beispiel 3

### Kupplung von o-Tolylmagnesiumchlorid mit p-Trifluormethylbrombenzol

Die Kupplung wurde analog zu der in Beispiel 1 beschriebenen Reaktion ausgeführt (0,001 mol-% PdCl₂(dppf)). Die Reaktion war nach nur 1 Stunde beendet; die Ausbeute betrug 96,1 %.

### Beispiel 4

### Kupplung von o-Tolyllithium mit p-Trifluormethylchlorbenzol

4'-Trifluormethyl-2-methylbiphenyl wurde durch Umsetzung von o-Tolyllithium in Toluol mit einem Äquivalent p-Trifluormethylchlorbenzol und 0,1 mol-% PdCl₂(PPh₃)₂ bei 50°C/0,5 h in einer Ausbeute von 78 % erhalten.

### Beispiel 5

### Kupplung von o-Tolylmagnesiumchlorid mit p-Trifluormethylphenyltriflat

4'-Trifluormethyl-2-methylbiphenyl konnte durch Umsetzung einer 26 gew.-%igen Lösung von o-Tolylmagnesiumchlorid in THF mit einem Äquivalent p-Trifluormethylphenyltriflat (erhältlich aus p-Trifluormethylphenol und Trifluormethansulfonsäureanhydrid oder Trifluormethansulfonylchlorid/Triethylamin/ Dichlormethan/0,1 % DMAP) und 0,1 mol-% PdCl₂(dppf) bei 50°C/0,5 h in einer Ausbeute von 95,5 % erhalten werden.

Beispiele zur Herstellung von 4'-Trifluormethylbiphenyl-2-carbonsäure

### Beispiel 6

### Oxidation mit Kaliumpermanganat in wässriger Lösung

Zu 75 ml einer 2 gew.-%igen wässrigen Kaliumpermanganat-Lösung wurden bei Raumtemperatur 1,0 g wasserfreies Natriumcarbonat und 1,13 g 4'-Trifluormethyl-2-methylbiphenyl gegeben. Nach 1,5-stündigem Rühren bei 100°C konnte nach Filtration des ausgefallenen Braunsteins, Ansäuern und Filtration des ausgefallenen Produkts 4'-Trifluormethylbiphenyl-2-carbonsäure in einer Ausbeute von 21 % erhalten werden.

### Beispiel 7

### Oxidation mit Kaliumpermanganat in wässriger Lösung mit Phasentransferkatalysator

Zu 75 ml einer 2 gew.-%igen wässrigen Kaliumpermanganat-Lösung wurden bei Raumtemperatur 1,0 g wasserfreies Natriumcarbonat, 0,2 g Benzyltributylammoniumchlorid und 1,13 g 4'-Trifluormethyl-2-methylbiphenyl gegeben. Nach 1,5-stündigem Rühren bei 100°C konnte nach Filtration des ausgefallenen Braunsteins, Ansäuern und Filtration des ausgefallenen Produkts 4'-Trifluormethylbiphenyl-2-carbonsäure in einer Ausbeute von 76 % erhalten werden.

### Beispiel 8

### Oxidation mit Luft

In einem 3,5 I-Autoklaven werden 177,2 g (0,75 Mol) 2-Methyl-4'-trifluormethylbiphenyl, 1511,2 g Essigsäure, 3,74 g (15,0 mMol) Cobalt(II)-acetat-tetrahydrat, 3,68 g (15,0 mMol) Mangan(II)-acetat-tetrahydrat und 3,09 g (30,0 mMol) Natriumbromid vorgelegt. Der Autoklav wird mit Stickstoff inertisiert und auf 160-165°C geheizt. Bei Erreichen dieser Temperatur werden ca. 450 l/h Luft eingeleitet und über eine Druckhalteeinrichtung ein Innendruck von 16-18 bar aufrecht erhalten. Man leitet ca. 30-40 Minuten Luft ein, inertisiert dann wieder durch Aufdrücken von Stickstoff und kühlt die Mischung ab. Die grünliche Lösung (wird beim Abkühlen orangerot) wird aus dem Autoklaven genommen und am Rotationsverdampfer auf 370 g eingeengt. Die dickflüssige Suspension wird abgenutscht und die Kristalle 3 mal mit je 50 g 75 %iger Essigsäure gewaschen. Man erhält 155 g 4'-Trifluormethylbiphenyl-2-carbonsäure (77,6 % der Theorie), die noch mit dem Fluorenon der Formel (V) verunreinigt ist.

### Beispiel 9

### Oxidation mit Luft

In einem 3,5 I-Autoklaven werden 177,2 g (0,75 Mol) 2-Methyl-4'-trifluormethylbiphenyl, 1350 g Essigsäure, 150 g Wasser, 3,74 g (15,0 mMol) Cobalt(II)-acetat-tetrahydrat, 3,68 g (15,0 mMol) Mangan(II)-acetat-tetrahydrat und 3,09 g (30,0 mMol) Natriumbromid vorgelegt. Der Autoklav wird mit Stickstoff inertisiert und auf 160-165°C geheizt. Bei Erreichen dieser Temperatur werden ca. 450 l/h Luft eingeleitet und über eine Druckhalteeinrichtung ein Innendruck von 16-18 bar aufrecht erhalten. Man leitet ca. 30-40 Minuten Luft ein, inertisiert dann wieder durch Aufdrücken von Stickstoff und kühlt die Mischung ab. Die grünliche Lösung (wird beim Abkühlen orangerot) wird aus dem Autoklaven genommen und am Rotationsverdampfer auf 350 g eingeengt. Die dickflüssige Suspension wird abgenutscht und die Kristalle 3 mal mit je 50 g 75 %iger Essigsäure gewaschen. Man erhält 180 g 4'-Trifluormethylbiphenyl-2'-carbonsäure (90,2 % der Theorie), die noch mit dem Fluorenon der Formel (V) verunreinigt ist.

### Beispiel 10

### Reinigung der Produkte aus Luftoxidation

136,5 g des Rohproduktes aus Beispiel 8 oder 9 werden in eine Mischung aus 100 g 33 %iger Natronlauge und 900 g Wasser eingetragen und 30 min gerührt. Man filtriert vom ungelösten Feststoff ab, wäscht den Rückstand mit Wasser und trocknet diesen.

| 2-Trifluormethyl-9-fluorenon | |
|---|---|
| Ausbeute | 15,5 g (gelbe Kristalle) |
| | 11,4 % der eingesetzten Menge |
| Schmelzpunkt | 129-133°C |

Das Filtrat wird mit Salzsäure versetzt. Der Niederschlag wird abgenutscht, gewaschen und getrocknet.

| 4'-Trifluormethyl-biphenyl-2-carbonsäure | |
|---|---|
| Ausbeute | 119,3 g (helle Kristalle) |
| | 87,4 % der eingesetzten Menge |
| Schmelzpunkt | 167-170°C (Lit.: 168-169 °C) |

## Patentansprüche

1. Verfahren zur Herstellung einer Verbindung der Formel (1) 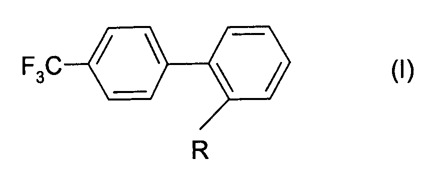 worin R die Bedeutung Methyl oder Carboxyl hat, dadurch gekennzeichnet, dass man ein ortho-Tolylmetallat der Formel (II) mit einer Verbindung der Formel (III) 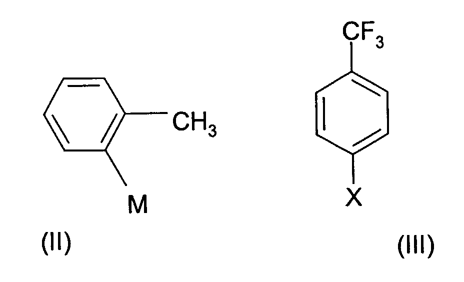 worin
M die Bedeutung -MgF, -MgCI, -MgBr, -Mgl, -Li, -ZnF, -ZnCI, -ZnBr oder -Znl und X die Bedeutung F, Cl, Br, l, N₂⁺, geradkettiges oder verzweigtes (C₁-C₂₀)-Alkoxy, Arylsulfonat oder Alkylsulfonat hat, in Gegenwart eines Ni-, Pd- oder Platinmetall-Katalysators zu einer Verbindung der Formel (I) mit R gleich CH₃ kuppelt, und gegebenenfalls die Verbindung der Formel (I) mit R gleich CH₃ zur Verbindung der Formel (I) mit R gleich Carboxyl oxidiert.

2. Verfahren nach Anspruch 1, dadurch gekennzeichnet, dass das o-Tolylmetallat, o-Tolylmagnesiumchlorid oder o-Tolylmagnesiumbromid ist.

3. Verfahren nach Anspruch 1 oder 2, dadurch gekennzeichnet, dass X die Bedeutung Cl, Br, Triflat, Tosylat, Nonaflat oder Mesylat hat.

4. Verfahren nach einem oder mehreren der Ansprüche 1 bis 3, dadurch gekennzeichnet, dass der Katalysator PdCl₂(dppf), PdCl₂(dppp), PdCl₂(dppe), PdCl₂(dppb), PdCl₂(PPh₃)₂, Pd(PPh₃)₄, Pd(OAc)₂, PdCl₂, PdBr₂ oder NiCl₂(PPh₃)₂ ist.

5. Verfahren nach einem oder mehreren der Ansprüche 1 bis 4, dadurch gekennzeichnet, dass der Katalysator in einer Menge von 10⁻⁵ bis 50 Mol-%, vorzugsweise von 10⁻⁴ bis 10 Mol-%, bezogen auf Mol Verbindung der Formel (III), eingesetzt wird.

6. Verfahren nach einem oder mehreren der Ansprüche 1 bis 5, dadurch gekennzeichnet, dass die Kupplung in einem Ether, Kohlenwasserstoff, halogeniertem Kohlenwasserstoff oder einer Mischung davon durchgeführt wird, vorzugsweise in THF oder THF/Toluol-Gemischen.

7. Verfahren nach einem oder mehreren der Ansprüche 1 bis 6, dadurch gekennzeichnet, dass die Kupplung bei einer Temperatur von 0 bis 180°C, vorzugsweise 30 bis 150°C, durchgeführt wird.

8. Verfahren nach einem oder mehreren der Ansprüche 1 bis 7, dadurch gekennzeichnet, dass die Oxidation mit Salpetersäure, einem Permanganat, einer Chrom(VI)-Verbindung, Sauerstoff oder Luft durchgeführt wird.

9. Verfahren nach einem oder mehreren der Ansprüche 1 bis 8, dadurch gekennzeichnet, dass die Oxidation mit Kaliumpermanganat in Gegenwart eines Phasentransferkatalysators durchgeführt wird.

10. Verfahren nach einem oder mehreren der Ansprüche 1 bis 8, dadurch gekennzeichnet, dass die Oxidation mit Luft in Gegenwart einer aliphatischen Carbonsäure und eines Schwermetallsalzes durchgeführt wird.
